Europäisches Patentamt

European Patent Office

Office européen des brevets

(19)

(11) Publication number: 0 349 258
A2

(12) **EUROPEAN PATENT APPLICATION**

(21) Application number: 89306496.4

(22) Date of filing: 27.06.89

(51) Int. Cl.⁴: G01N 33/577 , C12P 21/00 , C12N 15/00 , C12N 5/00 , //G01N33/574

(30) Priority: 28.06.88 JP 162222/88

(43) Date of publication of application: 03.01.90 Bulletin 90/01

(84) Designated Contracting States: CH DE FR GB IT LI SE

(71) Applicant: Shin-Etsu Chemical Co., Ltd.
6-1, Ohtemachi 2-chome
Chiyoda-ku Tokyo 100(JP)

(72) Inventor: Onodera, Kazukiyo
7-27-314, Hikawadai 2-chome Nerima-ku
Tokyo(JP)
Inventor: Obata, Ranko
1-599, Kosugi Jinya-Machi Nakahara-ku
Kawasaki-shi Kanagawa-ken(JP)
Inventor: Ito, Shinichi
Magnolia Court Woodside Grange Road N12
London(GB)

(74) Representative: Votier, Sidney David et al
CARPMAELS & RANSFORD 43, Bloomsbury
Square
London WC1A 2RA(GB)

(54) Method of determining the differentiation of a human T-cell.

(57) A method of determining the differentiation of a human T-cell comprising the step of applying a particular monoclonal antibody to said human T-cell. The monoclonal antibody has been produced by a hybridoma obtained by immunizing an animal with a Chinese hamster ovary cell which expresses at least one membrane protein encoded by human chromosome No. 21 and fusing an antibody-producing cell taken from said animal with a myeloma cell to form hybridomas, and has a characteristic that it specifically binds said Chinese hamster ovary cell but does not bind a normal Chinese hamster ovary cell. This method is useful in diagnosis of human leukemia.

EP 0 349 258 A2

## Method of determining the differentiation of a human T-cell

BACKGROUND OF THE INVENTION

1. Field of the invention

This invention relates to a method of determining the differentiation of a human T-cell.

2. Description of the Prior Art

Since the tumors of hemotopoietic organs such as myelocytic leukemia and leukemia-lymphoma of T-cell are markedly diversified, the therapy therefor are different depending on the type of each tumor. Accordingly, when the therapy is selected, it is necessary to learn the differentiation (i.e., the stage or degree of differentiation) of the tumor cell in question.

SUMMARY OF THE INVENTION

An object of this invention is to provide a novel method of ditermining the differentiation of a human T-cell which makes it possible to readily and accurately ditermine the differentiation.

The present inventors has discovered that a particular monoclonal antibody binds or does not bind a particular human T-cell depending on the differentiation of the human T-cell in question, and that, based on this fact, the monoclonal antibody is useful in determining the differentiation of human T-cells

Thus, this invention provides a method of determining the differentiation of a human T-cell, comprising the step of applying a monoclonal antibody

that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and

that specifically binds said Chinese hamster ovary cell that expresses at least one membrance protein encoded by human chromosome No. 21 but does not bind a normal Chinese hamster ovary cell,

to said human T-cell.

The monoclonal antibody used in this invention binds or does not bind a human leukemia-lymphoma cell line to T-cell in its own fashion or profile depending on the differentiation of the human T-cell leukemia cell. Therefore the method of this invention makes it possible to determine the differentiation of a human T-cell readily and accurately using one or more, preferably two or more, kinds of the monoclonal anitibody. The method of this invention is therefore useful to diagnosis of human leukemia.

DETAILED DESCRIPTION OF THE PREFERRED EMBODIMENTS

The monoclonal antibody used in this invention is disclosed in the Japanese Unexamined Patent Publication (KOKAI) No. 240,797/1988. This monoclonal antibody can be obtained by a process comprising the steps of immunizing an animal with a Chinese hamster ovary (CHO) cell into which human chromosome No. 21 has been introduced and which synthesizes a membrane protein encoded by human chromosome No. 21; fusing an antibody-producing cell taken from the animal with a myeloma cell to form hybridomas; subjecting the hybridomas to selection, screening and cloning to obtain a hybridoma producing a desired monoclonal antibody; and culturing the hybridoma to obtain such monoclonal antibody.

The above screening procedure is carried out using the characteristic of the monoclonal antibody used in this invention that the monoclonal antibody can bind the CHO cell used in the above immunization but does not bind normal CHO cells.

The CHO cell into which human chromosome NO. 21 has been introduced so that the cell expresses a membrane protein encoded by human chromosome No. 21 can be obtained by known cell fusion techniques. An example of such a CHO cell is a cell of the so-called 2Fu$^r$ line [see D. Patterson et al., Som. Cell Genet. 1; 91-110 (1975)].

Examples of animals which are suitable to be immunized include rodents like mouse and rat. Mice are usually used in this regard.

Route and schedule of administration of the antigen into the animal may variously be varied. According to a preferred administration method, for example, the antigen is first treated with mitomycin and intraperitoneally administered. Antibodies are produced in the spleen of the animal as an immunological response to administration to the antigen. As antibody-producing cells, spleen cells are usually used.

For the myeloma cell to be fused with the antibody-producing cells thus obtained, a cell derived from BALB/C mouse MOPC 21 myeloma (P3-NS1-AG4-1) can be used. The myeloma P3-NS1-AG4-1 is resistant to 8-azaguanine, and dies in a medium containing hypoxanthine-aminopterin-thymidine (HAT medium) because it lacks the enzyme hypoxanthine guanine phoshphoribosyltransferase. Therefore, use of P3-NS1-AG4-1 is convenient for selective culture of hybridomas to be obtained. Polyethylene glycol is typically used as a fusing agent for cell fusion. The above-mentioned cell fusion techniques, myeloma cells to be used, and the like are known [meyloma cells: Köhler et al., Eur. J. Immunol. 6: 292-295 (1976); fusing method: Köhler et al., ibid. 6: 511-516 (1976)].

The resulting hybridomas are screened to select those which can produce antibodies capable of binding the CHO cell used for the immunization stated above (i.e., the CHO cell into which human chromosome No. 21 has been introduced and expresses a membrane protein encoded by the human chromosome No. 21) but incapable of binding a normal CHO cell. The hybridomas thus screened are then cloned, whereby progeny of different hybridomas from respective phyletic lines are obtained. Monoclonal antibodies that can recognize a membrane protein chracteristic of cells where chromosome No. 21 is present in aberrant number can be synthesized and secreted by culturing the hybridomas to produce cell lines, i.e., by propagating each clone in a cell culture medium (in vitro culture) or in vivo. Each of the monoclonal antibodies thus produced can be recovered according to a method known in the art.

The monoclonal antibody used in this invention is also, as described in the Japanese Unexamined Patent Publication (KOKAI) No. 240,797/1988, characterized in that it specifically binds a human fibroblast cell with a membrane protein component encoded by chromosome No. 21 in an aberrant number but does not bind a normal human fibroblast cell.

It has also been found that the monoclonal antibody used in this invention specifically binds or does not bind a particular human T-cell depending on the differentiation of the human T-cell in question. The T-cell may be a normal T-cell or a leukemia-lymphoma cell of T-cell. Therefore, the method of this invention is useful in determining the differentiation of leukemia-lymphoma cell lines of T-cell.

The method of this invention can be conducted in practice by using any immunological test method, for example, immunofluorescence, an immunoadhesionhemocyte-agglutinating reaction method, radioimmunoassay or the like. In the utilization of immunofluorescence, for example, a human T-cell is treated with the monoclonal antibody, then reacted with an anti-mouse IgG labeled with a fluorescent dye such as FITC, and observed with a fluorescence microscope. The state of luminescence is then checked, and the differentiation of the human T-cell can be thereby easily determined.

The present invention will now be described in more detail with reference of Examples.

## EXAMPLES

### Preparation example (Preparation of Monoclonal Antibody)

(1) Procuction of antibodies

CHO cells (2Fu$^r$) into which human chromosome No. 21 had been introduced and which synthesized a membrane protein encoded by the chromosome No. 21 were treated with mitomycin C and then washed with PBS (phosphate buffer). A 2Fu$^r$ suspension of $5 \times 10^6$ cells/0.5 cc in PBS was intraperitoneally injected into a female BALB/C mouse (immunization). Two weeks thereafter, a 2Fu$^r$ suspension of $1 \times 10^7$ cells/0.5 cc in PBS, and further two weeks thereafter, 2Fu$^r$ suspension of $1 \times 10^7$ .cells/0.5 cc in PBS were injected in the same manner as above. Three days thereafter, the spleen of the mouse was taken out, finely pulverized in RPMI-1640 medium, subjected three times to centrifugation (1500 rpm) for washing, and resuspended in the medium.

Sample cultures of 2Fu$^r$ used here can be obtained upon written request to Shin-Etsu Chemical Co., Ltd., 6-1, Ohtemachi 2-chome, Chiyoda-ku, Tokyo, Japan.

3

(2) Cell fusion

1.1 x 10$^8$ cells of the spleen cell obtained in the above procedure and 1.0 x 10$^7$ cells of BALB/C mouse myeloma cell (P3-NS1-Ag4-1) once washed in advance with RPMI-1640 medium were mixed, and centrifuged at 1500 rpm for 5 minutes to prepare a pellet. Then, 1 ml of a solution obtained by dissolving polyethylene glycol (1540) in RPMI-1640 medium to 50 wt% was added to the pellet with stirring, RPMI-1640 medium was added thereto to the total volume of 10 ml, and the mixture was stirred for 6 minutes. The mixture was subjected to centrifugation as 1000 rpm for 5 minutes to remove polyethylene glycol, and the solid matters were resuspended in 40 ml of RPMI-1640 medium containing 20 vol% of fetal calf serum (FCS).

(3) Selection, screening and cloning

0.2 ml (5 x 10$^6$ cells) of the above suspension was placed in each of 192 wells of a 96 wells-plate for tissue culture, and half amount of the culture supernatant was replaced every 3 to 4 days with HAT medium (136.1 mg/ml hypoxanthine, 1.76 mg/ml aminopterine and 38.75 mg/ml thymidine). The culturing condition was a temperature of 37° C, a humidity of 100% and a $CO_2$ gas concentration of 7%. 10 days after the start of the culturing, hybridomas were observed in 119 wells (emergence rate: 62%). Culture supernatants in the 119 wells, which revealed positive results, were subjected to screening, by means of an enzyme-labeled antibody technique (ELISA) using 2Fu$^r$, to determine whether the supernatants contained antibodies. The test results indicated that 20 wells (10.6% of the total number) were positive.

Cells in each of the 20 wells were subjected to limiting dilution using HAT medium with supplementation of BALB/C mouse thymocyte (1.0 x 10$^7$ cells/ml), 0.2 ml portions of the resulting cell suspensions were placed in each well of a 96 wells-plate for tissue culture, and culturing was carried out. In this connection, hybridoma number per well and well number were 48 wells of 1 cell/well, 45 wells of 5 cells/well and 3 cells of 20 cells/well. Culturing conditions were the same as above. The culture supernatants were respectively subjected to the same ELISA as above-mentioned to screen those which are negative for the CHO cell and positive for 2Fu$^r$, and as the result desired clones were obtained from three wells. The cells in each of the three wells were recloned two times according to the limiting dilution method, and three kinds of resulting hybridomas were name OK-1, OK-2 and OK-3. Sample cultures of hybridomas OK-2 and OK-3 are available from FRI (Fermentation Research Institute Agency of Industrial Science and Technology), Ibaraki, Japan under the deposit Nos. FERM BP-1802 and FERM BP-1803, respectively.

It has been found by an enzyme-labeled antibody technique using an antimouse antibody that monoclonal antibodies produced from hybridomas OK-1, OK-2 and OK-3 belong to the classes IgM, IgG and IgM, respectively.

Furthermore, proteins to which the monoclonal antibodies produced by OK-1, OK-2 and OK-3 respectively recognize and bind are as follows.

- Monoclonal antibody produced by OK-1

This antibody recognizes four bans in Western blotting of human leukemia cell (TALL-1) protein.

- Monoclonal antibody procuded by OK-2

This antibody recognizes bands of 40 and 90 kilodaltons in Western blotting of TALL-1 protein.

- Monoclonal antibody procuced by OK-3

This antibody recognizes four bans of 86.4, 74.2, 61.4 and 36.4 kilodaltons in Western blotting of TALL-1 protein, and recognizes three bans of 89, 82 an 45 kilodaltons in Western blotting of 2Fu$^r$ protein.

Example

4

## Determination of the differentiation of a human T-cell by immunofluorescence

Seven types of leukemia-lymphoma cell lines of T-cell, NALM 16, MOLT-10, HPB-ALL, TALL-1, MOLT-4, SKW and MT-2 (see MAMMALIAN CELL CULTURE TECHNOLOGY, edited by M. Shikita et al. SOFT SCIENCE PUBLICATIONS, Tokyo, pp. 141-162, 1985) were separately cultured in RPMI-1640 medium containing 10% fetal calf serum. For each cell line, after the cultivation, $5 \times 10^6$ cells were washed by repeating three times the operations of suspending the cells in a phosphate buffered saline (PBS) containing 3% bovine serum albumine (BSA) and then centrifuging the suspension at 1,000 rpm, for five minutes. Each of three kinds of monoclonal antibodies obtained from the hybridomas OK-1, OK-2 and OK-3 in the afore-stated Preparation Example was diluted to 400-fold with PBS containing 0.02 M sodium azide and 3% BSA to give a monoclonal antibody solution. In 50 μl of the solution thus obtained was suspended the leukemia T-cell after washed as described above, and the suspension was left to react. After the reaction, the cells were washed in the same manner as described above.

After washed, the cells were suspended and allowed to react at room temperature for 30 minutes in an aqueous solution containing fluoresceine thiocyante (FITC) -bound anti-mouse IgG or IgM antibody which was prepared by dilution to 200-fold with PBS containing 3% BSA.

After the reaction, the cells were washed by centrifugation. Thereafter, the cells were suspended in a PBS containing 7% BSA and observed with a fluorescence microscope.

The results are given in Table 1. The results show that each of the three monoclonal antibodies produced by the hybridomas OK-1, OK-2 and OK-3, respectively, binds or does not bind a human leukemia-lymphoma cell line of T-cell in its own profile depending on the particular differentiation of the T-cell. Accordingly, from the binding profile of one or more monoconal antibodies, it is possible to determine the differentiation of the human leukemia-lymphoma cell line of T-cell.

Table 1

| Differentiation | 0 | I | II | III | | IV | V |
|---|---|---|---|---|---|---|---|
| Cell line | NALM-16 | MOLT-10 | HPB-ALL | TALL-1 | MOLT-4 | SKW | MT-2 |
| Monoclonal antibody | (nonT-nonB) | | | | | | |
| Produced by OK-1 | - | + | - | + | - | + | + |
| Produced by OK-2 | - | - | - | + | + | + | + |
| Produced by OK-3 | - | - | - | + | - | - | + |
| Note : | | | | | | | |
| +:Luminescence was observed <br> -:Luminescence was not observed | | | | | | | |

## Claims

1. A method of determining the differentiation of a human T-cell comprising the step of applying a monoclonal antibody
that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and
that specifically binds said Chinese hamster ovary cell that expresses at least one membrane protein encoded by human chromosome No. 21 but does not bind a normal Chinese hamster ovary cell,
to said human T-cell.

2. The method of Claim 1, wherein said monoclonal antibody is produced by a cell line OK-2 or OK-3.

3. For determining the differentiation of a human T-cell, a monoclonal antibody
that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and that specifically binds said Chinese hamster ovary cell that expresses at last one membrane protein encoded by human chromosome No. 21 but does not bind a normal Chinese hamster ovary cell.

4. The monoclonal antibody of Claim 3 which is produced by a cell line OK-2 or OK-3.